# EUROPEAN PATENT SPECIFICATION

(11) **EP 1 708 664 B1**
(45) Date of publication and mention of the grant of the patent: **21.03.2012**
(21) Application number: 04814434.9
(22) Date of filing: 14.12.2004
(51) Int. Cl.: A61F 13/15, A61F 13/42

(54) **ARTICLE FEATURING AN INTERIOR GRAPHIC**
ARTIKEL MIT EINER INNEREN GRAFIK
ARTICLES PRESENTANT UN MOTIF GRAPHIQUE INTERIEUR

(30) Priority: 29.12.2003 US 748411
(43) Date of publication of application: 11.10.2006
(73) Proprietor: KIMBERLY-CLARK WORLDWIDE, INC., Neenah, WI 54956 (US)
(72) Inventor: SOSALLA, Paula, M., Appleton, Wisconsin 54911 (US); BUSHMAN, Lisa, L., Kaukauna, Wisconsin 54130 (US)
(74) Representative: Mabey, Katherine Frances
(86) International application number: PCT/US2004/042247
(87) International publication number: WO 2005/065613

(56) References cited:
- WO-A-2004/006818
- US-A1- 2001 031 954
- US-A1- 2003 073 966
- US-A1- 2003 105 443

## Description

### FIELD OF THE INVENTION

This invention relates generally to training pants, and more particularly to such articles having interior graphics.

### BACKGROUND OF THE INVENTION

Absorbent articles such as diapers, training pants, incontinence garments, and the like often include a liquid-impermeable outercover, an absorbent body, and in some instances, graphics disposed on the exterior surface of the outercover. The graphics may provide a decorative feature, and particularly when used in connection with children's training pants, may be used to make the pants appear similar to conventional underwear. Further, the graphics may also be used to encourage training and/or be used to make the training experience more enjoyable and a generally positive experience. For example, the graphics may be used to allow the caregiver to interact with the wearer in the training setting.

Accordingly, the graphics may take various forms, such as in the form of a character, object and/or alphanumeric (e.g., numbers, words, phrases, instructions, etc.), and the like. Moreover, at least some of the graphics may be configured to be capable of appearing or disappearing when the article is exposed to liquid, such as urine. These graphics can alert the wearer and the caregiver to the occurrence of urine in the article (i.e., an "accident"), and can assist in the training process.

Nonetheless, such graphics, in certain circumstances, may not be completely satisfactory. For example, in some configurations, appearing or disappearing graphics visible on the exterior of the article may require a considerable amount of liquid, or multiple accidents, before the graphics are caused to appear or disappear. Additionally, graphics on the exterior of the article do not necessarily motivate the wearer to pull the article up and down for inspection, which can be a key training step.

US2003/105443 discloses an article having graphics printed on the outer cover thereof.

There is need, therefore, to provide a suitable training tool that can help notify the wearer of any accidents, even if the accident is relatively small in volume. In addition, there is a need for a training tool that encourages the wearer to practice pulling the article up and down.

### SUMMARY OF THE INVENTION

The present invention provides a training part in accordance with claim 1.

In one aspect, the present invention is directed to an absorbent article in the form of a training part defining a longitudinal direction and a lateral direction, an interior article surface and an exterior article surface opposite the interior article surface. The article includes an outercover defining an interior outercover surface and an exterior outercover surface opposite the interior outercover surface, and an absorbent body disposed on the interior outercover surface. The article also includes at least one interior graphic disposed on the interior article surface where the at least one interior graphic is an active graphic.

In embodiments, the article includes an outercover, a bodyside liner placed in superposed relationship with the outercover, and an absorbent body sandwiched between the outercover and the bodyside liner. The outercover is disposed toward the exterior article surface and the bodyside liner is disposed toward the interior article surface. The article may also include at least one interior graphic disposed on the interior article surface where the at least one interior graphic is a fading graphic.

In embodiments, the present invention may provide an absorbent article in the form of a training part defining a longitudinal direction and a lateral direction, an interior article surface and an exterior article surface opposite the interior article surface, a first waist region, a second waist region, and a crotch region connecting the first and second waist regions. The article may include side panels extending from and connecting the first and second waist regions in a pant configuration to form a waist opening and a pair of leg openings. In these embodiments, the article also includes an outercover defining an interior outercover surface and an exterior outercover surface opposite the interior outercover surface, and an absorbent body disposed on the interior outercover surface. The article further includes at least one interior graphic disposed on the interior article surface where the at least one interior graphic is an active graphic.

The above-mentioned and other aspects of the present invention will become more apparent, and the invention itself will be better understood by reference to the drawings and the following description of the drawings.

### BRIEF DESCRIPTION OF THE DRAWINGS

Figure 1 representatively illustrates a side view of a pair of training pants with a mechanical fastening system of the pants shown fastened on one side of the training pants and unfastened on the other side of the training pants;

Figure 2 representatively illustrates a plan view of the training pants of Figure 1 in an unfastened, stretched and laid flat condition, and showing the surface of the training pants that faces away from the wearer;

Figure 3 representatively illustrates a plan view similar to Figure 2, but showing the surface of the training pants that faces the wearer when worn, and with portions cut away to show underlying features and with one aspect of a graphic of the present invention applied to the pants;

Figure 4 representatively illustrates a plan view similar to Fig. 3 with another aspect of a graphic of the present invention applied to the pants; and

Figures 5A, 5B and 5C representatively illustrate various partial section views of the pants.

Corresponding reference characters indicate corresponding parts throughout the drawings.

### DETAILED DESCRIPTION

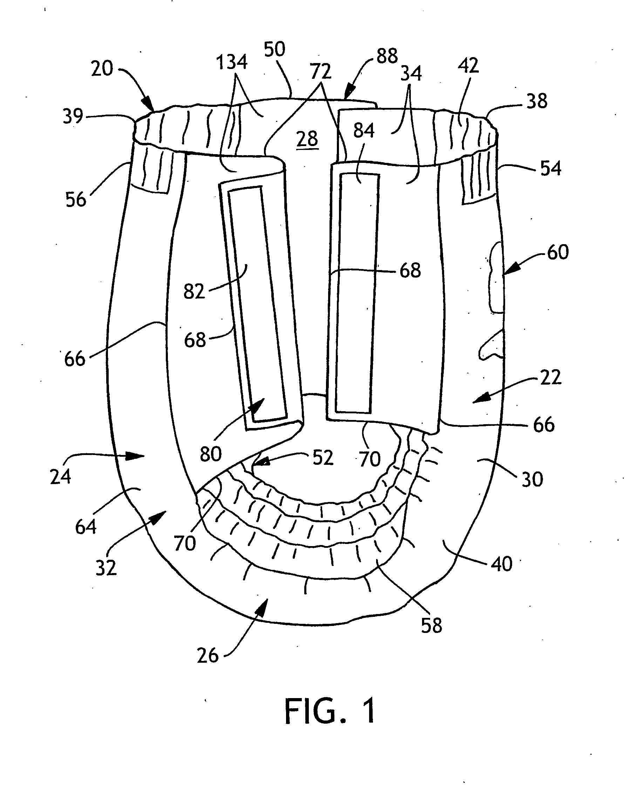

Referring now to the drawings and in particular to Fig. 1, a disposable article in the form of children's toilet training pants is indicated in its entirety by the reference numeral 20. The article may or may not be absorbent, which generally refers to absorbent particles that may be placed against or in proximity to the body of the wearer to absorb and/or retain various liquid waste discharged from the body. The term "disposable" as used herein refers to articles that are intended to be discarded after a limited period of use instead of being laundered or otherwise restored for reuse.

By way of illustration only, training pants suitable for use with the present invention and various materials and methods for constructing the training pants are disclosed in PCT Patent Application WO 00/37009 published June 29, 2000 by A. Fletcher et al; U.S. Patent 4,940,464 issued July 10, 1990 to Van Gompel et al.; U.S. Patent 5,766,389 issued June 16, 1998 to Brandon et al.; and U.S. Patent 6,645,190 issued November 11, 2003 to Olson et al.

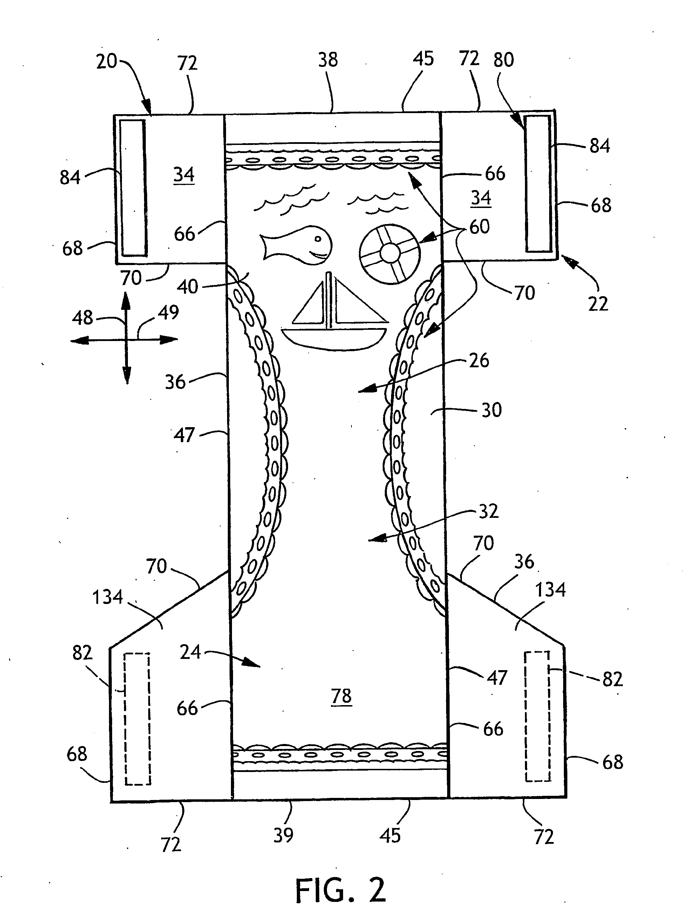

The training pant 20 is illustrated in Fig. 1 in a partially fastened condition and includes a first waist region and a second waist region, i.e., the front waist region 22, and the back waist region 24. The training pant 20 also includes a crotch region 26 interconnecting the front and back waist regions, an interior article surface 28 configured for a facing relationship with the wearer, and an exterior article surface 30 opposite the interior article surface. With additional reference to Figs. 2 - 4, the training pants 20 also has a pair of laterally opposite side edges 36 and a pair of longitudinally opposite waist edges, respectively designated front waist edge 38 and back waist edge 39.

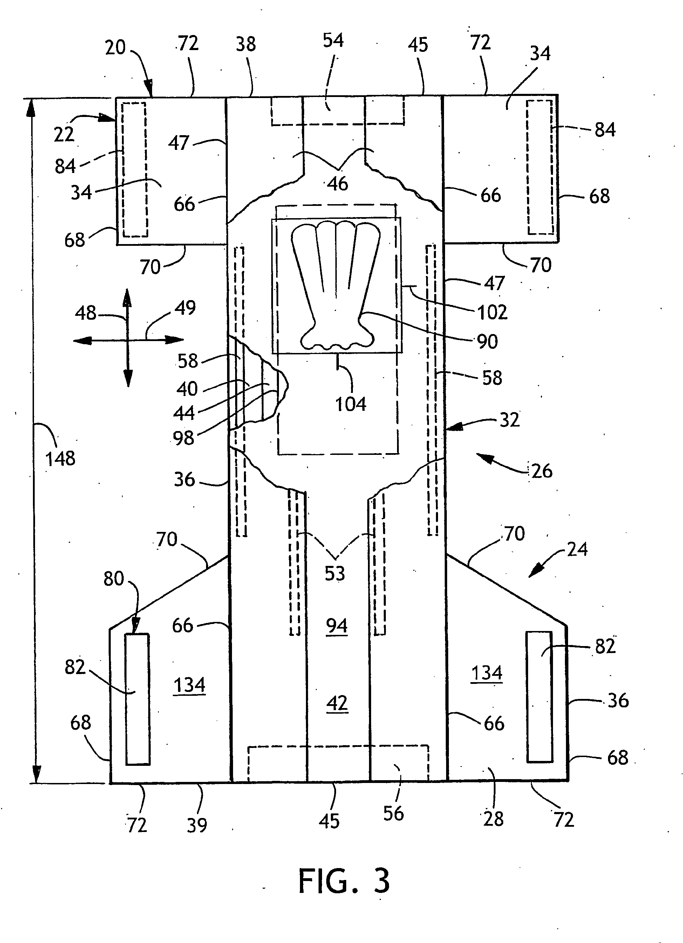

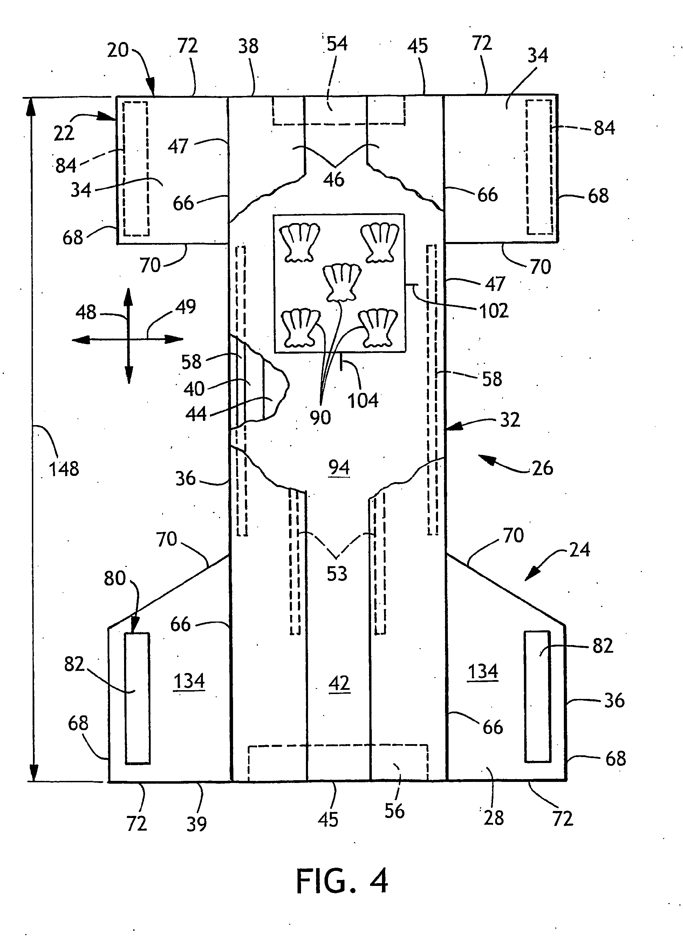

The illustrated pants 20 includes a central absorbent assembly, generally indicated at 32, which when laid flat can be rectangular or any other desired shape, a pair of laterally opposite front side panels 34 extending outward therefrom at the front waist region 22 and a pair of laterally opposite back side panels 134 extending outward therefrom at the back waist region 24. The absorbent assembly 32 and side panels 34, 134 may comprise two or more separate elements, as representatively illustrated in Figs. 1 - 4, or be integrally formed. The central absorbent assembly 32 of the illustrated aspect includes an outercover 40, a bodyside liner 42 (Figs. 1, 3 and 4) connected to the outercover in a superposed relation, an absorbent body 44 (Figs. 3 and 4) disposed between the outercover 40 and the bodyside liner 42, and a pair of containment flaps 46 (Figs. 3 and 4). The central absorbent assembly also has opposite ends 45 which can form portions of the front and back waist edges 38 and 39, and opposite side edges 47 which can form portions of the side edges 36 of the training pants 20 (Figs. 2 - 4). Integrally formed side panels 34, 134 and absorbent assembly 32 would comprise at least some common materials, such as the bodyside liner 42, flap composite, outercover 40, other materials and/or combinations thereof, and could define a one-piece elastic, stretchable, or nonstretchable pants. For further reference, arrows 48 and 49 depict the orientation of the longitudinal axis and the transverse or lateral axis, respectively, of the training pants 20 (Figs. 2 - 4). Likewise, arrow 148 indicates the article length in the longitudinal direction 48 of the pant 20 from the front waist edge 38 to the back waist edge 39 (Figs. 3 and 4).

With the training pants 20 in the fastened position as partially illustrated in Fig. 1, the front and back side panels 34, 134 are connected together by a fastening system 80 to define a three-dimensional pants configuration having a waist opening 50 and a pair of leg openings 52. The front waist region 22 comprises the portion of the training pants 20 which, when worn, is positioned on the front of the wearer while the back waist region 24 comprises the portion of the training pants which is positioned on the back of the wearer. The crotch region 26 of the training pants 20 comprises the portion of the training pants 20 which is positioned between the legs of the wearer and covers the lower torso of the wearer. The front and back side panels 34 and 134 comprise the portions of the training pants 20 which, when worn, are positioned on the hips of the wearer. The waist edges 38 and 39 of the training pants 20 are configured to encircle the waist of the wearer and together define the waist opening 50 (Fig. 1). Portions of the side edges 36 in the crotch region 26 generally define the leg openings 52.

The central absorbent assembly 32 is configured to contain and/or absorb exudates discharged from the wearer. For example, the containment flaps 46 are configured to provide a barrier to the transverse flow of body exudates. A flap elastic member 53 (Figs. 3 and 4) can be operatively joined with each containment flap 46 in any suitable manner as is well known in the art. The elasticized containment flaps 46 define a partially unattached edge which assumes an upright configuration in at least the crotch region 26 of the training pants 20 to form a seal against the wearer's body. The containment flaps 46 can be located along the side edges 36 of the pants 20, and can extend longitudinally along the entire length of the absorbent assembly 32 or may only extend partially along the length of the absorbent assembly. Suitable constructions and arrangements for the containment flaps 46 are generally well known to those skilled in the art and are described in U.S. Patent 4,704,116 issued November 3, 1987 to Enloe.

To further enhance containment and/or absorption of body exudates, the training pants 20 also suitably includes a front waist elastic member 54 (Figs. 1, 3 and 4), a rear waist elastic member 56, and leg elastic members 58, as are known to those skilled in the art. The waist elastic members 54 and 56 can be operatively joined to the outercover 40 and/or the bodyside liner 42 along the opposite waist edges 38 and 39, and can extend over part or all of the waist edges. The leg elastic members 58 can be operatively joined to the outercover 40 and/or the bodyside liner 42 along the opposite side edges 36 and positioned in the crotch region 26 of the training pants 20. The leg elastic members 58 can be longitudinally aligned along each side edge 47 of the absorbent assembly 32.

The flap elastic members 53, the waist elastic members 54 and 56, and the leg elastic members 58 can be formed of any suitable elastic material. As is well known to those skilled in the art, suitable elastic materials include sheets, strands or ribbons of natural rubber, synthetic rubber, or thermoplastic elastomeric polymers. The elastic materials can be stretched and adhered to a substrate, adhered to a gathered substrate, or adhered to a substrate and then elasticized or shrunk, for example with the application of heat, such that elastic retractive forces are imparted to the substrate. In one particular aspect, for example, the leg elastic members 58 comprise a plurality of dry-spun coalesced multifilament spandex elastomeric threads sold under the trade name LYCRA® and available from E. I. Du Pont de Nemours and Company, Wilmington, Delaware, U.S.A.

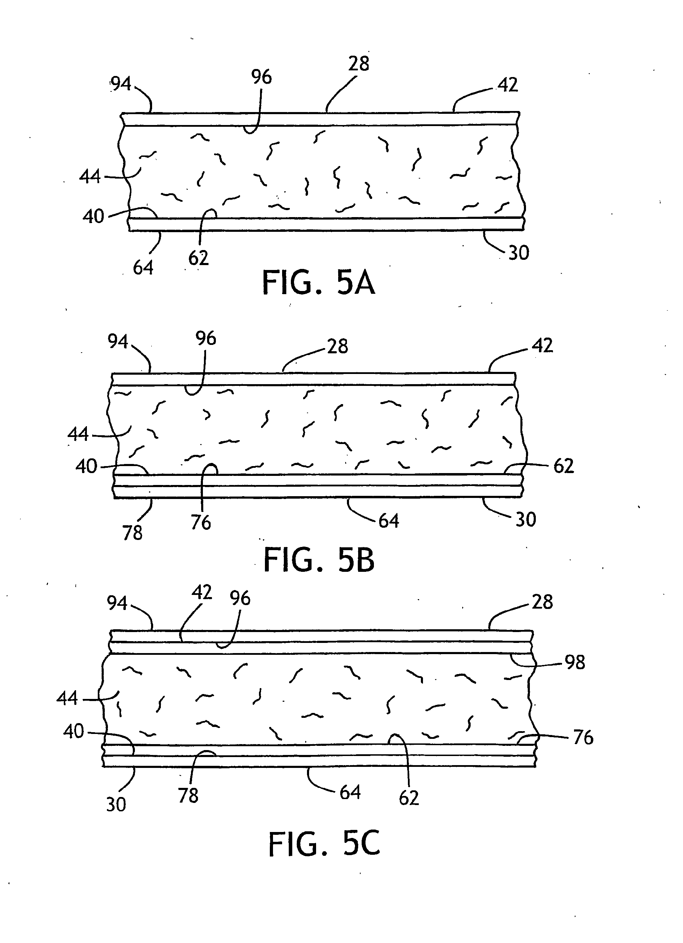

The outercover 40 of the present invention is disposed toward the exterior article surface 30. For example, the outercover 40 can define an exterior outercover surface 64 corresponding to the exterior article surface 30 of the training pant 20 and an opposite interior outercover surface 62. The outercover 40 desirably comprises a material that is substantially liquid impermeable. The outercover 40 can be a single layer of liquid impermeable material, but desirably comprises a multi-layered laminate structure in which at least one of the layers is liquid impermeable. For instance, as representatively illustrated in Figs. 5B and 5C, the outercover 40 can include a liquid permeable outer layer 78 and a liquid impermeable inner layer 76 that are suitably joined together by a laminate adhesive (not shown). Suitable laminate adhesives, which can be applied continuously or intermittently as beads, a spray, parallel swirls, or the like, can be obtained from Findley Adhesives, Inc., of Wauwatosa, Wisconsin, or from National Starch and Chemical Company, Bridgewater, New Jersey. The liquid permeable outer layer 78 can be any suitable material and desirably one that provides a generally cloth-like texture. One example of such a material is a 20 gsm (grams per square meter) spunbond polypropylene nonwoven web. The outer layer 78 can also be made of those materials of which liquid permeable bodyside liner 42 is made. While it is not a necessity for outer layer 78 to be liquid permeable, it is desired that it provides a relatively cloth-like texture to the wearer.

The inner layer 76 of the outercover 40 can be both liquid and vapor impermeable, or can be liquid impermeable and vapor permeable. The inner layer 76 is desirably manufactured from a thin plastic film, although other flexible liquid impermeable materials can also be used. The inner layer 76, or the liquid impermeable outercover 40 when a single layer, prevents waste material from wetting articles, such as bedsheets and clothing, as well as the wearer and caregiver. A suitable liquid impermeable film for use as liquid impermeable inner layer, or a single layer liquid impermeable outercover 40, is a 0.025 millimeter (1.0 mil) polyethylene film commercially available from Edison Plastics Company of South Plainfield, New Jersey. If the outercover 40 is a single layer of material, it can be embossed and/or matte finished to provide a more cloth-like appearance. As earlier mentioned, the liquid impermeable material can permit vapors to escape from the interior of the disposable absorbent article, while still preventing liquids from passing through the outercover 40. A suitable "breathable" material is composed of a microporous polymer film or a nonwoven fabric that has been coated or otherwise treated to impart a desired level of liquid impermeability. A suitable microporous film is a PMP-1 film material commercially available from Mitsui Toatsu Chemicals, Inc., Tokyo, Japan, or an XKO-8044 polyolefin film commercially available from 3M Company, Minneapolis, Minnesota.

The liquid permeable bodyside liner 42 is illustrated as being in superposed relationship with the outercover 40, with the outercover 40 and the bodyside liner 42 sandwiching the absorbent body 44. The bodyside liner 42 may, but need not have the same dimensions as the outercover 40. The bodyside liner 42 is disposed toward the interior article surface 28; for example, at least a portion of the bodyside liner 42 may provide the interior article surface 28. In particular, the bodyside liner 42 may define an interior liner surface 94 and an exterior liner surface 96 opposite the interior liner surface; accordingly, the interior liner surface 94 may provide at least a portion of the interior article surface 28.

The bodyside liner 42 is desirably compliant, soft feeling, and non-irritating to the child's skin. Further, the bodyside liner 42 can be less hydrophilic than the absorbent body 44, to present a relatively dry surface to the wearer and permit liquid to readily penetrate through its thickness. Alternatively, the bodyside liner 42 can be more hydrophilic or can have essentially the same affinity for moisture as the absorbent body 44 to present a relatively wet surface to the wearer to increase the sensation of being wet. This wet sensation can be useful as a training aid: The hydrophilic/hydrophobic properties can be varied across the length, width and depth of the bodyside liner 42 and absorbent body 44 to achieve the desired wetness sensation or leakage performance.

The bodyside liner 42 can be manufactured from a wide selection of web materials, such as synthetic fibers (for example, polyester or polypropylene fibers), natural fibers (for example, wood or cotton fibers), a combination of natural and synthetic fibers, porous foams, reticulated foams, apertured plastic films, or the like. Various woven and nonwoven fabrics can be used for the bodyside liner 42. For example, the bodyside liner can be composed of a meltblown or spunbonded web of polyolefin fibers. The bodyside liner can also be a bonded-carded web composed of natural and/or synthetic fibers. The bodyside liner can be composed of a substantially hydrophobic material, and the hydrophobic material can, optionally, be treated with a surfactant or otherwise processed to impart a desired level of wettability and hydrophilicity. For example, the material can be surface treated with about 0.45 weight percent of a surfactant mixture comprising Ahcovel N-62 from Hodgson Textile Chemicals of Mount Holly, North Carolina, U.S.A. and Glucopan 220UP from Henkel Corporation of Ambler, Pennsylvania in an active ratio of 3:1. The surfactant can be applied by any conventional means, such as spraying, printing, brush coating or the like. The surfactant can be applied to the entire bodyside liner 42 or can be selectively applied to particular sections of the bodyside liner, such as the medial section along the longitudinal center line.

A suitable liquid permeable bodyside liner 42 is a nonwoven bicomponent web having a basis weight of about 27 gsm. The nonwoven bicomponent can be a spunbond bicomponent web, or a bonded carded bicomponent web. Suitable bicomponent fibers include a polyethylene/polypropylene bicomponent fiber available from CHISSO Corporation, Osaka, Japan.

The absorbent body 44 (Figs. 3 - 5C) is sandwiched between the outercover 40 and the bodyside liner 42, which can be joined together by any suitable means such as adhesives, ultrasonic bonds, thermal bonds, or the like. In particular, the absorbent body 44 may be disposed on the interior surface of the outercover 40. As used herein, the term "disposed on" and variations thereof are intended to mean that one element can be integral with another element, or that one element can be a separate structure bonded to or placed with or placed near another element.

The absorbent body 44 can be any structure which is generally compressible, conformable, non-irritating to the child's skin, and capable of absorbing and retaining liquids and certain body wastes, and may be manufactured in a wide variety of sizes and shapes, and from a wide variety of liquid absorbent materials commonly used in the art. For example, the absorbent body 44 can suitably comprise a matrix of hydrophilic fibers, such as a web of cellulosic fluff, mixed with particles of a high-absorbency material commonly known as superabsorbent material. In a particular aspect, the absorbent body 44 comprises a matrix of cellulosic fluff, such as wood pulp fluff, and superabsorbent hydrogel-forming particles. The wood pulp fluff can be exchanged with synthetic, polymeric, meltblown fibers or short cut homofil bicomponent synthetic fibers and natural fibers. The superabsorbent particles can be substantially homogeneously mixed with the hydrophilic fibers or can be nonuniformly mixed. The fluff and superabsorbent particles can also be selectively placed into desired zones of the absorbent body 44 to better contain and absorb body exudates. The concentration of the superabsorbent particles can also vary through the thickness of the absorbent body 44. Alternatively, the absorbent body 44 can comprise a laminate of fibrous webs and superabsorbent material or other suitable means of maintaining a superabsorbent material in a localized area.

Suitable superabsorbent materials can be selected from natural, synthetic, and modified natural polymers and materials. The superabsorbent materials can be inorganic materials, such as silica gels, or organic compounds, such as crosslinked polymers, for example, sodium neutralized polyacrylic acid. Suitable superabsorbent materials are available from various commercial vendors, such as BASF Aktiengesellschaft located in Ludwigshafen, Germany, and Stockhausen GmbH & Co. KG, Krefeld, Germany. Typically, a superabsorbent material is capable of absorbing at least about 10 times its weight in water, and suitably is capable of absorbing more than about 25 times its weight in water.

In one aspect, the absorbent body 44 comprises a blend of wood pulp fluff and superabsorbent material. One preferred type of pulp is identified with the trade designation CR1654, available from U.S. Alliance, Childersburg, Alabama, U.S.A., and is a bleached, highly absorbent sulfate wood pulp containing primarily soft wood fibers and about 16 percent hardwood fibers. As a general rule, the superabsorbent material is present in the absorbent body 44 in an amount of from 0 to about 90 weight percent based on total weight of the absorbent assembly. The absorbent body 44 may or may not be wrapped or encompassed by a suitable tissue wrap that may help maintain the integrity and/or shape of the absorbent assembly.

The central absorbent assembly 32 can also incorporate other materials or components designed primarily to receive, temporarily store, and/or transport liquid along the mutually facing surface with the absorbent body 44, thereby maximizing the absorbent capacity of the absorbent assembly. One suitable additional component is commonly referred to as a surge layer and comprises a material having a basis weight of about 50 to about 120 grams per square meter, and more particularly comprises a through-air-bonded-carded web of a homogenous blend of 60 percent 3 denier type T-256 bicomponent fiber comprising a polyester core/polyethylene sheath and 40 percent 6 denier type T-295 polyester fiber, both commercially available from Kosa Corporation of Salisbury, North Carolina, U.S.A. As will be described in greater detail below, a component that may designed to receive, temporarily store and/or transport liquid, or provide a surge layer may be the interior graphic layer 98.

As noted previously, the training pants 20 may have front and back side panels 34 and 134 disposed on each side of the absorbent assembly 32. The side panels 34, 134 can be permanently bonded along seams 66 to the central absorbent assembly 32 in the respective front and back waist regions 22 and 24. More particularly, as seen best in Figs. 2-4, the front side panels 34 can be permanently bonded to and extend transversely outward beyond the side edges 47 of the absorbent assembly 32 in the front waist region 22, and the back side panels 134 can be permanently bonded to and extend transversely outward beyond the side edges of the absorbent assembly in the back waist region 24. The side panels 34 and 134 may be bonded to the absorbent assembly 32 using attachment means known to those skilled in the art such as adhesive, thermal or ultrasonic bonding. Alternatively, the side panels 34 and 134 can be formed as an integral portion of a component of the absorbent assembly 32. For example, the side panels can comprise a generally wider portion of the outercover 40, the bodyside liner 42, and/or another component of the absorbent assembly 32. The front and back side panels 34 and 134 can be permanently bonded together or be releasably connected with one another such as by the fastening system 80 of the illustrated aspect.

The front and back side panels 34, 134 each have an outer edge 68 spaced laterally from the seam 66, a leg end edge 70 disposed toward the longitudinal center of the training pants 20, and a waist end edge 72 disposed toward a longitudinal end of the training pants. The leg end edge 70 and waist end edge 72 extend from the side edges 47 of the absorbent assembly 32 to the outer edges 68. The leg end edges 70 of the side panels 34 and 134 form part of the side edges 36 of the training pants 20. The leg end edges 70 of the illustrated aspect are suitably curved and/or angled relative to the transverse axis 49 to provide a better fit around the wearer's legs. However, it is understood that only one of the leg end edges 70 may be curved or angled, such as the leg end edge of the back waist region 24, or neither of the leg end edges may be curved or angled, without departing from the scope of this invention. The waist end edges 72 are suitably parallel to the transverse axis 49. The waist end edges 72 of the front side panels 34 form part of the front waist edge 38 of the training pants 20, and the waist end edges 72 of the back side panels 134 form part of the back waist edge 39 of the pants.

The side panels 34, 134 suitably, although not necessarily, comprise an elastic material capable of stretching in a direction generally parallel to the transverse axis 49 of the training pants 20. Suitable elastic materials, as well as one process of incorporating elastic side panels into training pants, are described in the following U.S. Patents: 4,940,464 issued July 10, 1990 to Van Gompel et al.; 5,224,405 issued July 6, 1993 to Pohjola; 5,104,116 issued April 14, 1992 to Pohjola; and 5,046,272 issued September 10, 1991 to Vogt et al. In particular aspects, the elastic material may comprise a stretch-thermal laminate (STL), a neck-bonded laminate (NBL), a reversibly necked laminate, or a stretch-bonded laminate (SBL) material. Methods of making such materials are well known to those skilled in the art and described in U.S. Patent 4,663,220 issued May 5, 1987 to Wisneski et al.; U.S. Patent 5,226,992 issued July 13, 1993 to Morman; European Patent Application No. EP 0 217 032 published on April 8, 1987 in the name of Taylor et al.; and PCT application WO 01/88245 in the name of Welch et. Alternatively, the side panel material may comprise other woven or nonwoven materials, such as those described above as being suitable for the outercover 40 or bodyside liner 42; mechanically pre-strained composites; or stretchable but inelastic materials.

The fastening system 80 comprises laterally opposite first fastening components 82 adapted for refastenable engagement to corresponding second fastening components 84. In one aspect, a front or outer surface of each of the fastening components 82, 84 comprises a plurality of engaging elements. The engaging elements of the first fastening components 82 are adapted to repeatedly engage and disengage corresponding engaging elements of the second fastening components 84 to releasably secure the pants 20 in its three-dimensional configuration. A suitable fastening system is described in U.S. Patent 6,645,190 to Olson, et al.

As representatively illustrated in Fig. 1, when the fastening components 82, 84 are releasably engaged, the side edges 36 of the training pants 20 in the crotch region 26 define the leg openings 52, and the waist edges 38 and 39 including the waist end edges 72 of the side panels 34, 134 define the waist opening 50. For improved formation of the leg openings 52, it can be desirable in some aspects for the front side panels 34 to be longitudinally spaced from the back side panels 134 as illustrated in Figs. 2 and 3. For example, the front side panels 34 can be longitudinally spaced from the back side panels 134 by a distance equal to about 20 percent or greater, particularly from about 20 to about 60 percent, and more particularly from about 35 to about 50 percent, of the overall length of the pants 20.

When engaged, the fastening components 82, 84 of the illustrated aspect define refastenable engagement seams 88 (Fig. 1) which desirably although not necessarily extend substantially the entire distance between the waist opening 50 and the leg openings 52.

The training pants 20 of the various aspects of the present invention further include at least one interior graphic 90 disposed on the interior article surface 28 of the pant 20. For example, as representatively illustrated in Fig. 3, the pant 20 may include a single interior graphic 90 on the interior article surface 28. Alternatively, as representatively illustrated in Fig. 4, the pant 20 may include a plurality of interior graphics 90 on the interior article surface. The interior graphic 90 may include, but are not limited to, scenes, characters, animals, objects, alphanumerics such as numbers, letters, words, phrases and the like. In particular aspects, the interior graphic 90 may also be gender specific; that is, the interior graphic 90 may be a graphic that may be generally considered to be of interest to boys or to girls.

The interior graphic 90 can be formed or applied directly or indirectly to any surface of the pant 20 such that the interior graphic 90 is visible from the interior article surface 28. For example, the interior graphic 90 may be applied directly or indirectly to any surface of the liner 42 such as the interior liner surface 94 or the exterior liner surface 96, formed or applied between the liner 42 and the absorbent body 44, formed or applied to a substrate that is placed with or near the liner 42, such as an interior graphic layer 98, formed or applied within a layer of the liner 42 or another substrate, or other variations or combinations thereof. In one aspect, and as will be discussed in greater detail below, the interior graphic 90 can be printed, sprayed, or otherwise applied directly on a surface 94 or 96 of the liner 42. In a particular aspect, the interior graphic 90 may advantageously be applied on the interior liner surface 94. In such an arrangement, the materials that provide the interior graphic 90 are less likely to transfer onto the skin of the wearer.

In other aspects, the interior graphic 90 can be applied to a layer placed with or near the liner 42, such as on an interior graphic layer 98. As mentioned above, the interior graphic layer may be a substrate associated with the absorbent body 44, such as another absorbent layer, a tissue layer, a liquid handling layer, a liquid transfer layer or a surge layer. Alternatively, the interior graphic layer may be a tissue material, liquid handling layers, absorbent layers, or the like. For example, the interior graphic layer 98 may be provided by a tissue including natural fiber material such as any type of wood pulp. Alternatively, the interior graphic layer can also be made from an uncreped through air-dried (UCTAD) tissue material that is known in the art. The interior graphic layer 98 may suitably provide a more uniform surface upon which the interior graphic may be applied, resulting in a more consistent, sharp and discernable graphic.

Various placements of the interior graphic 90 may be better understood with reference to the partial section views of pants 20 that are shown in Figures 5A, 5B and 5C. As mentioned above, the various layers of the illustrated embodiments can be secured together using adhesives, thermal bonds, mechanical bonds, or other means known to those skilled in the art.

Figure 5A illustrates a partial section view of an absorbent article having an absorbent body 44 sandwiched between an outercover 40 and a bodyside liner 42. The illustrated outer cover 40 is a single layer having an interior outercover surface 62 and an opposite exterior outercover surface 64, which also may provide at least a portion of the exterior article surface 30. In addition, the illustrated liner 42 is a single layer having an exterior liner surface 96 and an interior liner surface 94, which also may provide at least a portion of the interior article surface 28. The interior graphics 90 can be disposed on the liner 42, which includes either surface 94 or 96 of the liner, on the surface of the absorbent body 44 that faces the liner 42, or between the absorbent body 44 and the liner 42.

Figure 5B illustrates a partial section view of another pant 20 having an absorbent body 44 sandwiched between an outercover 40 and a bodyside liner 42. The illustrated outercover 40 is a two-layer composite comprising an outercover outer layer 78 and an outercover inner layer 76. The outercover 40 has an interior outercover surface 62 and an opposite exterior outercover surface 64, which also may provide at least a portion of the exterior article surface 30. In addition, the illustrated liner 42 is a single layer having an exterior liner surface 96 and an interior liner surface 94, which also may provide at least a portion of the interior article surface 28. The interior graphics 90 can be disposed on the liner 42, which includes either surface 94 or 96 of the liner, on the surface of the absorbent body 44 that faces the liner 42, or between the absorbent body 44 and the liner 42.

Figure 5C illustrates a partial section view of a further aspect having an absorbent body 44 sandwiched between an outercover 40 and a bodyside liner 42. The pant 20 also includes an interior graphic layer 98 sandwiched between the absorbent body 44 and the liner 42. The illustrated outercover 40 is a two-layer composite comprising an outercover outer layer 78 and an outercover inner layer 76, but could alternatively be a single layer. The outercover 40 has an interior outercover surface 62 and an opposite exterior outercover surface 64, which also may provide at least a portion of the exterior article surface 30. In addition, the illustrated liner 42 is a single layer having an exterior liner surface 96 and an interior liner surface 94, which also may provide at least a portion of the interior article surface 28. The interior graphics 90 can be disposed on the liner 42, which includes either surface 94 or 96 of the liner, on the surface of the absorbent body 44 that faces the liner 42, or between the absorbent body 44 and the liner 42. The internal graphic 90 could also be disposed on either surface of the interior graphic layer 98 that is placed with or placed near the liner 42, between the interior graphic layer 98 and the liner 42 or between the interior graphic layer 98 and the absorbent body 44. Various other locations for the interior graphics can be suitable where different configurations of the absorbent body 44 and liner 42 are employed, including but not limited to within a layer of the absorbent body 44, liner 42, or interior graphic layer 98.

The interior graphics 90 of the present invention include active graphics, and may include combinations of active and permanent graphics. At least one of the interior graphics 90 is an active graphic. As used herein, the term "active graphic" refers to an appearing graphic, a fading graphic, or a combination of appearing and fading graphics. The term "appearing graphic" is used herein to refer to a graphic that becomes visible or becomes significantly more visible when exposed to urine. Conversely, the term "fading graphic" is used herein to refer to a graphic that becomes invisible or significantly less visible when exposed to urine. For example, when the wearer wets the training pant 20, liquid is communicated to the active graphic, whereupon the active graphic either dissolves, changes color, appears, or the like. Where appearing graphics are employed, the situation would work in reverse.

In one aspect, fading graphics of the present invention may be formed from an ink that is soluble in aqueous solutions such as urine. As such, the ink can be positioned in the pant 20 so that it becomes wet and dissolves when the product is insulted with liquid. Once dissolved, the ink washes away from the interior article surface 28. Thus, the ink may be obscured by the liner 42, absorbent body 44 or other layers that may be adjacent the liner 42 and/or absorbent body 44. As a result, the fading graphic seems to disappear from view.

Suitable urine-soluble inks are available from a variety of commercial vendors, such as Sun Chemical Corp. of Philadelphia, Pennsylvania, USA under the trade designation AQUA DESTRUCT. Particular urine-soluble compositions are disclosed in U.S. Patent 4,022,211 issued May 10, 1977 to Timmons et al. The ink color can be selected to provide a pleasing appearance and graphic impact, including fading rapidly upon contact with liquid. In particular aspects, and to facilitate rapid fading, the fading graphics can comprise line drawings having a line width of from about 1 to about 5 millimeters.

Alternatively, the active graphic can also comprise a fading or an appearing graphic which is formed from a composition such as an ink or adhesive that changes color when exposed to an aqueous solution such as urine. A color change composition can be adapted to blend in with a background or surrounding color, either before or after exposure to the aqueous solution. Suitable compositions of this color-change type are available from a variety of commercial vendors, such as a pH-change/color-change hot melt adhesive available from Findley Adhesives, Inc. of Wauwatosa, Wisconsin, USA. Alternatively, the active graphic can comprise pH sensitive inks, fugitive inks, colored absorbent particles, hydratable salts, moisture sensitive films, enzymes, heat sensitive inks and dyes, or the like.

In contrast to active graphics, the term "permanent graphic" is used herein to refer to a graphic that does not substantially change its degree of visibility when the absorbent article is insulted with urine in simulated use conditions. The change in visibility of a graphic or a portion of a graphic can be determined based on a person's observation of the graphic before and after the article containing the graphic is exposed to liquid. For purposes hereof, an article is exposed to liquid by immersing the article completely in an aqueous solution containing 0.9 weight percent sodium chloride, used at room temperature (≅23° C), for a period of twenty minutes. After 20 minutes the product is removed from the aqueous solution and placed on a TEFLON™ coated fiberglass screen having 0.25 inch openings, which is commercially available from Taconic Plastics Inc., Petersberg, New York, USA, which in turn is placed on a vacuum box and covered with a flexible rubber dam material. A vacuum of 3.5 kilopascals (0.5 pounds per square inch) is drawn in the vacuum box for a period of 5 minutes, after which the article is removed and observed. The person with normal or corrected vision of 20-20 should make the observations from a distance of 1 meter in an environment providing 30 footcandles (320 Lux) of illumination. Changes in the visibility of the graphic should be identified, and distinguished where necessary from changes in the color of other materials such as fluff pulp within an absorbent assembly. Desirably, the permanent graphic can be configured so that the entire graphic also does not substantially change its appearance, size or shape when the product is insulted with liquid or exposed to the environment.

In use, the active graphic can appear or fade when an accident occurs and urine comes into contact with the active graphic. In particular, the active graphic appears or fades in about 3 minutes or less, more particularly in about 1 minute or less, and still more particularly in about 20 seconds or less, when the absorbent article is insulted with 200 milliliters or more of urine, suitably when the absorbent article is insulted with about 40 to about 60 milliliters or more of urine, and more suitably when the absorbent article is insulted about 10 milliliters or more of urine.

Accordingly, as the pant 20 of the present invention includes an interior graphic 90 that is an active graphic, it can assist in the toilet training of the wearer. For example, the interior graphic 90 can encourage the wearer to pull the pant 20 up and down to inspect the graphic 90, an activity which is a key to toilet training and requires a relatively high level of coordination. In addition, as can be readily appreciated an active interior graphic 90 may encourage the wearer to refrain from wetting the pant 20 in order to keep the graphic 90 in place. Further, the interior graphic 90 can provide the wearer with a feeling of "ownership" over the graphic, as the graphic is intended primarily for their viewing. Still further, an active interior graphic 90 is more sensitive to even small accidents than other forms of active graphics due to their proximity to the target area. As such, active internal graphics can be particularly effective in late stage training.

Therefore, in order to provide a proper focus on the interior graphic 90, the interior graphic may be particularly positioned within the pant 20. Moreover, in configurations where the interior graphic 90 is an active graphic, it may be positioned in a gender specific target zone for urination within the product; such positioning can increase the likelihood that the interior graphic 90 will be activated by an insult. As such, in one aspect, at least a portion of the interior graphic 90 can be spaced from the front waist edge 38 in the longitudinal direction 48 by between 25 % and 50 % of the article length 148. In another aspect, at least a portion of the interior graphic 90 can be spaced from the front waist edge 38 in the longitudinal direction 48 by between 35 % and 60 % of the article length 148.

Moreover, each interior graphic 90 may define a total graphic area. The total graphic area may be calculated by multiplying the largest dimension of the interior graphic 90 in the longitudinal direction 48 (the interior graphic length, indicated at 102) by the largest dimension of the interior graphic 90 in the lateral direction 49 (the interior graphic width, indicated at 104) (Fig. 3). Therefore, in one aspect, the interior graphic 90 area may be at least 25 square cm. In another aspect, the interior graphic area may be at least 45 square cm. Alternatively, as representatively illustrated in Fig. 4 and as mentioned above, the pant 20 may include a plurality of interior graphics 90. Thus, the plurality of interior graphics 90 may, in total, define a total graphic area. The total graphic area may be calculated by multiplying the interior graphic length of the plurality of interior graphics in the longitudinal direction 48, indicated at 102, by the interior graphic width of the plurality of graphics in the lateral direction 49, indicated at 104 (Fig. 3). The plurality of interior graphics 90 may define a total graphic area of at least 25 square cm. Such total graphic areas as described above suitably draws the attention of the wearer and can therefore act as a more meaningful training aid.

The training pant 20 of the various aspects of the present invention may further include at least one exterior graphic 60 disposed on the exterior article surface 30. Suitably, the pant 20 may include a plurality of exterior graphics 60. The exterior graphics 60 may include, but are not limited to, scenes, characters, animals, objects, alphanumerics such as numbers, letters, words, phrases and the like, highlighting or emphasizing leg and waist openings 52, 50 in order to make product shaping more evident or visible to the user; highlighting or emphasizing areas of the product to simulate functional components such as elastic leg bands, elastic waistbands, simulated "fly openings" for boys, ruffles for girls; highlighting areas of the product to change the appearance of the size of the product; registering wetness indicators, temperature indicators, and the like in the product; registering a back label, or a front label, in the product; and registering written instructions at a desired location in the product.

The exterior graphics 60 can be formed on or applied to the outer cover 40 or another substrate bonded to or placed with or placed near the outer cover 40 by any suitable technique. The exterior graphics 60 are suitably registered with other components of the absorbent article during manufacture such that the exterior graphics 60 are positioned in the desired regions of the product.

The exterior graphics 60 may be active graphics, permanent graphics, or combinations thereof. In particular aspects, at least one of the exterior graphics is an active graphic, and more particularly a fading graphic. Exterior graphics 60 suitable for use with the present invention are described in U.S. Patent No. 6,297,424 issued October 2, 2001 to Olson, et al. and 6,307,119 issued October 23, 2001 to Cammarota et al.

The exterior graphics 60 of the present invention may also be configured to define a graphic theme. It should be noted that in order to establish a graphic theme, not every exterior graphic 60 need be directly related to the graphic theme, however, it can be appreciated that to effectively establish a graphic theme, it is desirable to have at least half of the exterior graphics 60 contribute to defining the graphic theme, more desirably the majority of the exterior graphics 60 contribute to defining the graphic theme, and still more desirably substantially all of the exterior graphics 60 contribute to defining the graphic theme. Similarly, it can be appreciated that where certain exterior graphics 60 are not contributing to defining the theme of the other exterior graphics 60, it can be most effective to at least have the non-contributing exterior graphics 60 be neutral or not in opposition toward the graphic theme.

The exterior graphics 60 may define a graphic theme when the subject matter of one exterior graphic 60 is the same as or is associated with the subject matter of another exterior graphic 60. For example, the exterior graphics 60 may be related by a unifying subject or common story line, which could be generally known through books, movies, common children's activities, or other sources to provide a graphic theme. By way of example, two objects are considered the same as or associated in subject matter where the images are identical; separately illustrate different sizes, shapes, colors of a common object; each illustrate one and the other of two objects that are commonly associated with one another, such as the moon and stars, a body of water and water toys, a sandbox and suitable toys, a baseball bat and ball, a barn and animals, or the like; illustrate different items used in a particular activity, such as a sporting activity, a gardening activity or the like; jointly illustrate geometrically mating or engaging elements such as a triangle and a triangularly-shaped aperture, or two halves of a zipper; each illustrate one part of a multipart picture; or the like. Similarly, two text messages are considered related in subject matter where the messages: are identical; jointly form a sentence, thought, or action such as "jump" and "up"; each refer to one and the other of two items that are commonly associated with one another, such as "bat" and "ball," "Big" and "Kid," "Big" and "Girl," or "Big" and "Boy"; jointly present a question and answer; or the like. Likewise, a text message and a pictorial image are considered to be related in subject matter where the text names, defines or describes the image; or the like.

Conversely, and by way of illustration and without wishing to be limited to the enumerated examples, two objects are considered unrelated in subject matter where the images: illustrate items that are neither identical nor illustrate two objects that are not commonly associated with one another, such as an animal and a building block, a jump rope and a flower, a car and a star, a letter of the alphabet and a water toy, a fish and an apple, illustrate items used in unrelated activities, such as items used in sporting activities and items used in gardening activities, or other unrelated activities; or the like. Similarly, two text messages are considered unrelated in subject matter where the messages: are neither identical nor jointly form a sentence, thought, or action; refer to two items that are not commonly associated with one another, such as "ball" and "flower," "fish" and "pencil," "car" and "ghost," or other such unrelated words; or the like. Likewise, a text message and a pictorial image are considered to be unrelated in subject matter where the text does not name, define, describe or otherwise relate to the image.

Thus, the interior graphic 90 may be related or unrelated to a graphic theme that may be defined by the exterior graphics 60. Suitably, the interior graphic 90 related to the graphic theme. In particular, and without wishing to be limited to the specific embodiments listed, suitable examples of a graphic theme with a related interior graphic 90 can include: the exterior graphics 60 being a racquet, bat, glove, other sporting equipment or the like and the interior graphic 90 comprising balls, or being related sporting equipment or the like; the exterior graphics 60 being a butterfly net or the like and the interior graphics 90 comprising butterflies or the like; the exterior graphics 60 being a fish, a boat or the like and the interior graphic 90 being a shell, water toys or the like (Figs. 3 - 4); the exterior graphics 60 being flowers, plants, gardening tools or the like and the interior graphic 90 comprising flowers or plants; the exterior graphics 60 being a specific environment such as a barn, silo, tractor or the like and the interior graphic 90 comprising cows, chickens, sheep, or the like which are specifically adapted to the environment; the exterior graphic 60 being a telescope, stars, planets or the like and the interior graphic 90 being rockets, spaceships or flying saucers.

In aspects where the interior graphic 90 is related to a theme established by the exterior graphics 60, it provides an opportunity for the wearer and the caregiver to interact and can improve the toilet training experience. For example, in one aspect the theme established by the exterior graphics 60 can include graphic that cannot complete some action or observation after a fading interior graphic 90 has disappeared. This facilitates nonthreatening and gentle communications between the wearer and caregiver when the child has had an accident and wet his or her pants. A caregiver might take a positive approach: "Try not to wet in your pants so the bear at the beach still has his sand toys to play with." Significantly this can be used as a motivational basis for teaching the child that it is within their control to permit the activity to continue for as long as the child can go without wetting his or her pants.

As mentioned above, the exterior graphics 60 and the interior graphics 90 may be disposed on the pant 20 using a variety of methods. For example, the graphics 60 and 90 may suitably be disposed on the pants 20 by being imprinted thereon using a flexographic printing process. Flexographic printing is a conventional printing technique which uses flexible, raised rubber or photopolymer plates to carry an inked image to a substrate, such as a bodyside liner 42, an interior graphic layer 98, or the outercover 40 of the pants 20. As an example, flexographic printing apparatus are shown and/or described in U.S. Patent Nos. 5,458,590 (Schleinz et al.); 5,566,616 (Schleinz et al.); U.S. 2003/0019374A1 (Harte); and 4,896,600 (Rogge et al.).

As noted previously, liquid soluble inks can be used to form the active graphics. It is theorized that the migration of the dissolved inks away from the surface upon which they are printed (for example, a surface of the liner 42, the absorbent body 44, or the interior graphic layer 98, or combinations thereof) and into the absorbent body 44 can improve the fading or disappearing quality of the active graphics. To enhance this effect, the liner 42. and/or the interior graphic layer 98 need not be bonded to the absorbent body 44. Alternatively, the liner 42 and/or the interior graphic layer 98 can be attached to the absorbent body 44 and to each other in a windowpane design, whereby the location of the active graphic 90 is not bonded to the absorbent body and the regions of the liner and/or the interior graphic layer 98 surrounding the active graphic are bonded. One suitable method and apparatus for adhesively bonding in a windowpane design is disclosed in U.S. Patent 5,683,752 issued November 4, 1997 to Popp et al.

As various changes could be made in the above constructions and methods, without departing from the scope of the invention, it is intended that all matter contained in the above description and shown in the accompanying drawings shall be interpreted as illustrative and not in a limiting sense.

When introducing elements of the invention or the preferred aspect(s) thereof, the articles "a", "an", "the" and "said" are intended to mean that there are one or more of the elements. The terms "comprising", "including" and "having" are intended to be inclusive and mean that there may be additional elements other than the listed elements.

## Claims

1. A training pant (20) defining a longitudinal direction (48) and a lateral direction (49), an interior article surface (28) configured for a facing relationship with a wearer and an exterior article surface (30) opposite said interior article surface (28), said training pant comprising:
an outercover (40) defining an interior outercover surface (62), and an exterior outercover surface (64) opposite said interior outercover surface (62); and
an absorbent body (44) disposed on said interior outercover surface (62); and
**characterised in that** said training pant further comprises at least one interior graphic (90) disposed on said interior article surface (28) so as to be visible from the interior article surface (28), wherein said at least one interior graphic (90) is an active graphic, the active graphic being an appearing graphic that becomes visible or becomes significantly more visible when exposed to urine, or a fading graphic that become invisible or significantly less visible when exposed to urine, or a combination of appearing and fading graphics.

2. The training pant of claim 1 comprising a first waist region (22), a second waist region (24), and a crotch region (26) connecting the first and second waist regions (22, 24); and
side panels (34, 134) extending from and connecting said first and second waist regions (22, 24) in a pant configuration to form a waist opening (50) and a pair of leg openings (52).

3. The training pant as claimed in any one of the preceding claims further comprising a bodyside liner (42) placed in superposed relationship with said interior outercover surface (62), wherein said absorbent body (44) is sandwiched between said outercover (40) and said bodyside liner (42), and wherein said interior article surface (28) is provided at least in part by said bodyside liner.

4. The training pant of claim 3 wherein said bodyside liner (42) defines an interior liner surface (94) and an exterior liner surface (96) opposite said interior liner surface, and wherein said interior graphic (90) is applied on said exterior liner surface.

5. The training pant of claim 3 further comprising an interior graphic layer (98) sandwiched between said absorbent body (44) and said bodyside liner (42), and wherein said interior graphic (90) is applied on said graphic layer (98).

6. The training pant of claim 5 wherein said interior graphic layer (98) is a surge layer.

7. The training pant as claimed in any one of the preceding claims further defining a front waist edge (38), a back waist edge (39), and an article length in said longitudinal direction, wherein said interior graphic (90) is spaced from said front waist edge in said longitudinal direction by between 25 % and 50 % of said article length.

8. The training pant as claimed in any one of claims 1 to 6 further defining a front waist edge (38), a back waist edge (39), and an article length in said longitudinal direction, wherein said interior graphic (90) is spaced from said front waist edge in said longitudinal direction by between 35 % and 60 % of said article length.

9. The training pant as claimed in any one of the preceding claims wherein said at least one interior graphic (90) defines a total interior graphic area and wherein said total interior graphic area is at least 25 square cm.

10. The training pant as claimed in any one of claims 1 to 8 wherein said at least one interior graphic (90) defines a total interior graphic area and wherein said total interior graphic area is at least 45 square cm.

11. The training pant as claimed in any one of claims 1 to 8 further comprising a plurality of interior graphics (90).

12. The training pant of claim 11 wherein said plurality of interior graphics (90) defines a total interior graphic area and wherein said total interior graphic area is at least 25 square cm.

13. The training pant as claimed in any one of the preceding claims further comprising at least one exterior graphic (60) disposed on said exterior article surface (30).

14. The training pant of claim 13 wherein said at least one exterior graphic (60) comprises a plurality of exterior graphics, and wherein at least one of said plurality of exterior graphics is an active graphic, the active graphic being an appearing graphic that becomes visible or becomes significantly more visible when exposed to urine, or a fading graphic that become invisible or significantly less visible when exposed to urine, or a combination of appearing and fading graphics.

15. The training pant as claimed in any one of the preceding claims wherein said active interior graphic (90) is a fading graphic, and is formed from ink that is soluble in urine.

## Patentansprüche

1. Trainingshöschen (20), welches eine Längsrichtung (48) und eine Querrichtung (49), eine innere Artikeloberfläche (28), die eingerichtet ist, einem Träger zugewandt zu sein, und eine äußere Artikeloberfläche (30) gegenüber der inneren Artikeloberfläche (28) definiert, wobei das Trainingshöschen umfasst:
eine Außenhülle (40), die eine innere Außenhüllenoberfläche (62) und eine äußere Außenhüllenoberfläche (64) gegenüber der inneren Außenhüllenoberfläche (62) definiert; und
einen absorptionsfähigen Körper (44), der auf der inneren Außenhüllenoberfläche (62) angeordnet ist; und
**dadurch gekennzeichnet, dass** das Trainingshöschen des Weiteren mindestens ein innere Graphik (90) umfasst, die auf der inneren Artikeloberfläche (28) angebracht ist, so dass sie von der inneren Artikeloberfläche (28) aus sichtbar ist, wobei die mindestens eine innere Graphik (90) eine aktive Graphik ist, wobei die aktive Graphik eine erscheinende Graphik ist, die sichtbar wird oder signifikant sichtbarer wird wenn sie Urin ausgesetzt ist, oder eine verschwindende Graphik ist, die unsichtbar wird oder signifikant weniger sichtbar wird, wenn sie Urin ausgesetzt ist, oder eine Kombination aus erscheinenden und verschwindenden Graphiken ist.

2. Trainingshöschen gemäß Anspruch 1, welches einen ersten Taillenbereich (22), einen zweiten Taillenbereich (24) und einen Schrittbereich (26) umfasst, der den ersten und zweiten Taillenbereich (22, 24) verbindet; und
welches Seitenbahnen (34, 134) umfasst, die sich von dem ersten und zweiten Taillenbereich (22, 24) erstrecken und diese in einer Höschenkonfiguration verbinden, um eine Taillenöffnung (50) und ein Paar von Beinöffnungen (52) zu bilden.

3. Trainingshöschen gemäß einem der vorherigen Ansprüche, welches des Weiteren eine körperseitige Auskleidung (42) umfasst, welche in überlagernder Beziehung mit der inneren Außenhüllenoberfläche (62) angeordnet ist, wobei der absorptionsfähige Körper (44) zwischen der Außenhülle (40) und der körperseitigen Auskleidung (42) angeordnet ist, und wobei die innere Artikeloberfläche (28) zumindest zum Teil durch die körperseitige Auskleidung bereitgestellt wird.

4. Trainingshöschen gemäß Anspruch 3, wobei die körperseitige Auskleidung (42) eine innere Auskleidungsoberfläche (94) und eine äußere Auskleidungsoberfläche (96) gegenüber der inneren Auskleidungsoberfläche definiert, und wobei die innere Graphik (90) auf die äußere Auskleidungsoberfläche aufgebracht ist.

5. Trainingshöschen gemäß Anspruch 3, welches des Weiteren eine innere Graphikschicht (98) umfasst, die zwischen dem absorptionsfähigen Körper (44) und der körperseitigen Auskleidung (42) angeordnet ist, und wobei die innere Graphik (90) auf die Graphikschicht (98) aufgebracht ist.

6. Trainingshöschen gemäß Anspruch 5, wobei die innere Graphikschicht (98) eine Durchflussschicht ist.

7. Trainingshöschen gemäß einem der vorherigen Ansprüche, welches des Weiteren eine vordere Taillenkante (38), eine hintere Taillenkante (39) und eine Artikellänge in der Längsrichtung definiert, wobei die innere Graphik (90) von der vorderen Taillenkante in der Längsrichtung um zwischen 25% und 50% der Artikellänge beabstandet ist.

8. Trainingshöschen gemäß einem der Ansprüche 1 bis 6, welches des Weiteren eine vordere Taillenkante (38), eine hintere Taillenkante (39) und eine Artikellänge in der Längsrichtung definiert, wobei die innere Graphik (90) von der vorderen Taillenkante in der Längsrichtung um zwischen 35% und 60% der Artikellänge beabstandet ist.

9. Trainingshöschen gemäß einem der vorherigen Ansprüche, wobei die mindestens eine innere Graphik (90) eine Gesamtfläche der inneren Graphik definiert und wobei die Gesamtfläche der inneren Graphik mindestens 25 Quadratzentimeter beträgt.

10. Trainingshöschen gemäß einem Ansprüche 1 bis 8, wobei die mindestens eine innere Graphik (90) eine Gesamtfläche der inneren Graphik definiert und wobei die Gesamtfläche der inneren Graphik mindestens 45 Quadratzentimeter beträgt.

11. Trainingshöschen gemäß einem der Ansprüche 1 bis 8, welches des Weiteren eine Vielzahl von inneren Graphiken (90) umfasst.

12. Trainingshöschen gemäß Anspruch 11, wobei die Vielzahl von inneren Graphiken (90) eine Gesamtfläche der inneren Graphiken definiert und wobei die Gesamtfläche der inneren Graphiken mindestens 25 Quadratzentimeter beträgt.

13. Trainingshöschen gemäß einem der vorherigen Ansprüche, welches des Weiteren mindestens eine äußere Graphik (60) umfasst, die auf der äußeren Artikeloberfläche (30) angeordnet ist.

14. Trainingshöschen gemäß Anspruch 13, wobei die mindestens eine äußere Graphik (60) eine Vielzahl von äußeren Graphiken umfasst, und wobei mindestens eine der Vielzahl von äußeren Graphiken eine aktive Graphik ist, wobei die aktive Graphik eine erscheinende Graphik ist, die sichtbar wird oder signifikant sichtbarer wird wenn sie Urin ausgesetzt ist, oder eine verschwindende Graphik ist, die unsichtbar wird oder signifikant weniger sichtbar wird, wenn sie Urin ausgesetzt ist, oder eine Kombination aus erscheinenden und verschwindenden Graphiken ist.

15. Trainingshöschen gemäß einem der vorherigen Ansprüche, wobei die aktive innere Graphik (90) eine verschwindende Graphik ist und aus einem Farbstoff gebildet ist, welcher in Urin löslich ist.

## Revendications

1. Couche-culotte (20) délimitant une direction longitudinale (48) et une direction latérale (49), une surface d'article intérieure (28) configurée de façon à faire face à une personne portant la couche-culotte et une surface d'article extérieure (30) opposée à ladite surface d'article intérieure (28), ladite couche-culotte comprenant :
une enveloppe (40) délimitant une surface d'enveloppe intérieure (62), et une surface d'enveloppe extérieure (64) opposée à ladite surface d'enveloppe intérieure (62) ; et
un corps absorbant (44) disposé sur ladite surface d'enveloppe intérieure (62) ;
et
**caractérisée en ce que** ladite couche-culotte comprend en outre au moins un graphique intérieur (90) disposé sur ladite surface d'article intérieure (28) de façon à être visible à partir de la surface d'article intérieure (28), ledit au moins un graphique intérieur (90) étant un graphique actif, le graphique actif étant un graphique apparaissant qui devient visible ou sensiblement plus visible quand il est exposé à de l'urine, ou un graphique disparaissant qui devient invisible ou sensiblement moins visible quand il est exposé à de l'urine, ou une combinaison de graphiques apparaissants et de graphiques disparaissants.

2. Couche-culotte selon la revendication 1, comprenant une première région de ceinture (22), une deuxième région de ceinture (24), et une région d'entrejambes (26) raccordant la première région de ceinture et la deuxième région de ceinture (22, 24) ; et
des panneaux latéraux (34, 134) qui s'étendent à partir de, et raccordent, ladite première région de ceinture et ladite deuxième région de ceinture (22, 24) dans une configuration correspondant à une culotte pour former une ouverture pour la taille (50) et une paire d'ouvertures pour les jambes (52).

3. Couche-culotte selon l'une quelconque des revendications précédentes, comprenant en outre une doublure faisant face au corps (42) positionnée de façon à être superposée avec ladite surface d'enveloppe intérieure (62), dans laquelle ledit corps absorbant (44) est positionné en sandwich entre ladite enveloppe (40) et ladite doublure faisant face au corps (42), et dans laquelle ladite surface d'article intérieure (28) est formée au moins en partie par ladite doublure faisant face au corps.

4. Couche-culotte selon la revendication 3, dans laquelle ladite doublure faisant face au corps (42) délimite une surface de doublure intérieure (94) et une surface de doublure extérieure (96) opposée à ladite surface de doublure intérieure, et dans laquelle ledit graphique intérieur (90) est appliqué sur ladite surface de doublure extérieure.

5. Couche-culotte selon la revendication 3, comprenant en outre une couche graphique intérieure (98) positionnée en sandwich entre ledit corps absorbant (44) et ladite doublure faisant face au corps (42), et dans laquelle ledit graphique intérieur (90) est appliqué sur ladite couche graphique (98).

6. Couche-culotte selon la revendication 5, dans laquelle ladite couche graphique intérieure (98) est une couche d'absorption rapide.

7. Couche-culotte selon l'une quelconque des revendications précédentes, délimitant en outre un bord de ceinture avant (38), un bord de ceinture arrière (39), et une longueur d'article dans ladite direction longitudinale, dans laquelle ledit graphique intérieur (90) est espacé dudit bord de ceinture avant dans ladite direction longitudinale dans une mesure correspondant à 25 % à 50 % de ladite longueur de l'article.

8. Couche-culotte selon l'une quelconque des revendications 1 à 6, délimitant en outre un bord de ceinture avant (38), un bord de ceinture arrière (39), et une longueur d'article dans ladite direction longitudinale, dans laquelle ledit graphique intérieur (90) est espacé dudit bord de ceinture avant dans ladite direction longitudinale dans une mesure correspondant à 35 % à 60 % de ladite longueur de l'article.

9. Couche-culotte selon l'une quelconque des revendications précédentes, dans laquelle ledit au moins un graphique intérieur (90) délimite une surface graphique intérieure totale et dans laquelle ladite surface graphique intérieure totale est d'au moins 25 cm².

10. Couche-culotte selon l'une quelconque des revendications 1 à 8, dans laquelle ledit au moins un graphique intérieur (90) délimite une surface graphique intérieure totale et dans laquelle ladite surface graphique intérieure totale est d'au moins 45 cm².

11. Couche-culotte selon l'une quelconque des revendications 1 à 8, comprenant en outre une pluralité de graphiques intérieurs (90).

12. Couche-culotte selon la revendication 11, dans laquelle ladite pluralité de graphiques intérieurs (90) délimite une surface graphique intérieure totale et dans laquelle ladite surface graphique intérieure totale est d'au moins 25 cm².

13. Couche-culotte selon l'une quelconque des revendications précédentes, comprenant en outre au moins un graphique extérieur (60) disposé sur ladite surface d'article extérieure (30).

14. Couche-culotte selon la revendication 13, dans laquelle ledit au moins un graphique extérieur (60) comprend une pluralité de graphiques extérieurs, et dans laquelle au moins l'un de ladite pluralité de graphiques extérieurs est un graphique actif, le graphique actif étant un graphique apparaissant qui devient visible ou sensiblement plus visible quand il est exposé à de l'urine, ou un graphique disparaissant qui devient invisible ou sensiblement moins visible quand il est exposé à de l'urine, ou une combinaison de graphiques apparaissants et de graphiques disparaissants.

15. Couche-culotte selon l'une quelconque des revendications précédentes, dans laquelle ledit graphique intérieur actif (90) est un graphique disparaissant, et est formé d'une encre qui est soluble dans l'urine.
